# EUROPEAN PATENT APPLICATION

(11) **EP 4 818 146 A1**
(43) Date of publication of application: **07.10.2026**
(21) Application number: 24898030.2
(22) Date of filing: 22.11.2024
(51) Int. Cl.: C07C 29/20, C07C 35/12, B01J 37/02, B01J 23/755

(54) **METHOD FOR PREPARING MENTHOL**

(30) Priority: 29.11.2023 KR 20230169286
(71) Applicant: Hanwha Solutions Corporation, Jung-gu Seoul 04541 (KR)
(72) Inventor: KIM, Youngjin, Daejeon 34128 (KR); DO, Seung Hoe, Daejeon 34128 (KR)
(74) Representative: Berggren Oy
(86) International application number: PCT/KR2024/018646
(87) International publication number: WO 2025/116434

(57) **Abstract**

The present disclosure relates to a method for preparing menthol, the method comprising a step of preparing menthol by a reduction reaction of thymol in the presence of an ammonia-treated metal-supported catalyst and hydrogen. The method for preparing menthol according to an aspect can satisfy both an excellent reaction rate and menthol selectivity.

## Description

### Technical Field

The present disclosure relates to a method of preparing menthol from thymol.

### Background Art

Menthol (2-Isopropyl-5-methylcyclohexanol) is a material that is useful in various industries such as medicine, cosmetics, and food. Menthol is a compound with three chiral centers, including four isomers of D/L-menthol, D/L-isomenthol, D/L-neomenthol, and D/L-neoisomenthol, and D/L-menthol is widely used in industry.

In current industry, a hydrogenation reaction of thymol (2-isopropyl-5-methylphenol), which is easy to commercialize, is mainly used as a method of preparing menthol, and because the hydrogenation reaction of thymol produces a complex mixture of the four isomers and menthane, which is a hydrocarbon byproduct, improving the selectivity of menthol remains a challenge that needs to be solved. Various research has been conducted to improve the selectivity of menthol, but there are limitations such as a process time being excessively long and a conversion rate being low due to a multi-step process or a method of reducing a reaction rate. In addition, even though methods of increasing the activity of the hydrogenation reaction was applied, excessive byproducts were produced, or the selectivity of menthol, which is a target product, was lowered or maintained.

Therefore, there is a need for a new technology capable of simultaneously satisfying the selectivity of menthol, the reaction rate, and a byproduct generation inhibition effect.

### Disclosure of Invention

### Technical Problem

An aspect of the present disclosure relates to a method of preparing menthol that may satisfy both an excellent reaction rate and an excellent menthol selectivity.

### Solution to Problem

An aspect of the present disclosure provides a method of preparing menthol including preparing the menthol by a reduction reaction of thymol in the presence of an ammonia-treated metal-supported catalyst and hydrogen.

The metal-supported catalyst may include a nickel-supported catalyst including one or more active metals selected from nickel and nickel oxide.

The metal-supported catalyst may include a silica support.

Treating the metal-supported catalyst with ammonia may be performed with ammonia gas at a temperature of 300 °C or less.

The reduction reaction may be performed under solvent-free conditions.

The reduction reaction may be performed under conditions of 1 bar to 100 bar of hydrogen and at a temperature of 150 °C to 200 °C.

The reduction reaction may be performed for 0.5 hours to 5 hours.

The amount of the metal-supported catalyst used may be 0.01 % by weight to 10 % by weight with respect to a total weight of menthol produced.

The conversion rate of the thymol may be 95 % or more.

The yield of a menthol isomer mixture may be 95 % or more.

The menthol isomer mixture may include menthol, isomenthol, neomenthol, and neoisomenthol.

The selectivity of menthol with respect to the menthol isomer mixture may be 50 % or more.

### Advantageous Effects of Invention

The method of preparing menthol according to an aspect may satisfy all of the excellent reaction rate, a menthol selectivity, and a byproduct generation inhibition effect even under solvent-free conditions. Specifically, the method of preparing menthol according to an aspect may effectively suppress hydrocarbon byproducts generated during a reduction process of thymol while having an excellent reaction rate even at a low temperature and low catalyst usage, and may improve the selectivity of menthol compared to menthol isomers.

In addition, the process may be further simplified by using a small amount of catalyst under non-solvent conditions according to an aspect, and menthol may be prepared at a high yield even under mild reaction conditions, and thus, productivity may be significantly improved.

### Best Mode for Carrying out the Invention

All terms used herein have the same meaning as commonly understood by one of ordinary skill in the art to which the present disclosure belongs. The terms used herein are intended to effectively describe specific embodiments and should not be construed as limiting the present disclosure.

As used herein, the singular forms are intended to include the plural forms as well, unless the context clearly indicates otherwise.

Throughout the specification, the terms 'comprises,' 'includes,' 'contains,' or 'has' do not exclude other components but mean that it may further include other components, unless specifically stated to the contrary, and do not exclude elements, materials, or processes that are not further listed.

A numerical range used herein includes the lower and upper limits, all values within the range, increments logically derived from the form and width of the defined range, all doubly limited values, and all possible combinations of the upper and lower limits of numerical ranges limited in different forms. Unless otherwise defined herein, values outside the numerical range that may occur due to experimental error or rounding of values are also intended to be included within the defined numerical range.

Unless otherwise defined herein, "about" may be considered to be within 30 %, 25 %, 20 %, 15 %, 10 %, or 5 % of a stated value.

Hereinafter, the present disclosure will be described in detail. However, this is merely an example, and the present disclosure is not limited to the specific embodiments described as examples.

Although the preparation of menthol through the hydrogenation reaction of thymol (2-isopropyl-5-methylphenol) is a widely used method in the current industry, the hydrogenation reaction of thymol produces menthol isomeric composite mixtures and hydrocarbon byproducts, which causes a limitation in low selectivity of menthol. Accordingly, various researches have been conducted to improve the selectivity of menthol, but commercialization has not been easy because a process time is excessively long and a conversion rate is low due to a multi-step process or a method of reducing a reaction rate. In addition, even though a method of increasing the activity of the hydrogenation reaction is applied, there are limitations such as excessive production of byproducts.

Accordingly, the present disclosure provides a method of preparing menthol that may ensure an excellent reaction rate and simultaneously satisfy both an excellent menthol selectivity and a byproduct generation inhibition effect.

The method of preparing menthol according an aspect may include preparing the menthol by a reduction reaction of thymol in the presence of an ammonia-treated metal-supported catalyst and hydrogen.

Specifically, the method of preparing menthol according to an aspect may include (a) ammonia-treating a metal-supported catalyst; (b) obtaining menthol by a hydrogenation reaction (reduction reaction) of thymol in the presence of the ammonia-treated metal-supported catalyst and hydrogen.

The ammonia-treating may include treating an ammonia aqueous solution (NH₄OH), vapor of the ammonia aqueous solution, or ammonia gas (NH₃).

The temperature in the ammonia-treating is not particularly limited, but may be, for example, 300 °C or less, or 10 °C to 300 °C, or 10 °C to 200 °C, or 10 °C to 100 °C, or 20 °C to 100 °C, or 20 °C to 80 °C.

The metal-supported catalyst according to an aspect may be formed by supporting an active metal on a support to form a composite. The active metal may be, for example, one or two or more selected from ruthenium, iron, nickel, cobalt, platinum, palladium, vanadium, copper, chromium, tungsten, molybdenum, iridium, rhodium, zirconium, cobalt, zinc, calcium, and oxides thereof. Specifically, the metal-supported catalyst according to an aspect may be a nickel-supported catalyst including at least one active metal selected from nickel (Ni) and nickel oxide (NiO).

The support may be one or two or more selected from silica, alumina, magnesium chloride, calcium chloride, booxite, zeolite, magnesium oxide, zirconium oxide, titanium oxide, boron trioxide, calcium oxide, zinc oxide, barium oxide, and thorium oxide, and specifically, silica. Specifically, the metal-supported catalyst according to an aspect may be a nickel-silica-supported catalyst in which an active metal including nickel, nickel oxide, or a combination thereof is supported on a silica support to form a composite.

For example, the metal-supported catalyst may include 5 to 50 parts by weight, 5 to 40 parts by weight, or 10 to 40 parts by weight of the support with respect to 100 parts by weight of the active metal. Specifically, when the metal-supported catalyst according to an aspect is a nickel-silica-supported catalyst in which an active metal including nickel and nickel oxide is supported on a silica support to form a composite, the metal-supported catalyst may include 50 wt% to 70 wt% of nickel, 10 wt% to 30 wt% of nickel oxide, and 50 wt% to 60 wt% of silica with respect to the total weight of the catalyst, and may specifically include 50 wt% to 60 wt% of nickel, 15 wt% to 25 wt% of nickel oxide, and 15 wt% to 25 wt% of silica.

The metal-supported catalyst according to an aspect may further include one or more promotors, and when the metal-supported catalyst further includes a promotor, the promotor may be included in an amount of 0.1 to 3.0 parts by weight with respect to 100 parts by weight of the active metal, and may further improve catalytic activity.

In addition, the method of preparing menthol according to an aspect may be performed under solvent-free conditions.

That is, the method of preparing menthol according to an aspect may satisfy all of an excellent reaction rate, a menthol selectivity, and a byproduct generation inhibition effect even under solvent-free conditions. Specifically, the method of preparing menthol according to an aspect may have an excellent reaction rate even at a low temperature and low catalyst usage, implement a thymol conversion rate close to 100 % in a short time, effectively suppress the production of byproducts, and implement a high menthol selectivity with respect to menthol isomers.

For example, the reduction reaction according to an aspect may be performed under conditions of 1 bar to 100 bar of hydrogen and a temperature of 100 °C to 200 °C, or 1 bar to 50 bar of hydrogen and a temperature of 150 °C to 200 °C, or 1 bar to 30 bar of hydrogen and a temperature of 160 °C to 190 °C.

For example, the amount of the metal-supported catalyst used may be 0.01 wt% to 20 wt%, or 0.01 wt% to 15 wt%, or 0.01 wt% to 10 wt%, or 0.1 wt% to 10 wt%, or 2 wt% to 10 wt%, with respect to the total weight of a produced menthol isomer mixture.

For example, the reduction reaction according to an aspect may be performed for 0.5 to 5 hours, 0.5 to 3 hours, or 0.1 to 2 hours under the above-described conditions.

For example, in the method of preparing menthol according to an aspect, the conversion rate of thymol ((thymol addition amount- thymol residual amount after completion of the reaction)/thymol input amount x100) may be 90 % or more, or 95 % or more, or 98 % or more, specifically 100 %.

For example, in the method of preparing menthol according to an aspect, the yield of the menthol isomer mixture may be 80 % or more, or 85 % or more, 90 % or more, or 95% or more, and in this regard, the upper limit thereof may be 99 % or less, or 98 % or less.

The menthol isomer mixture may include menthol, isomenthol, neomenthol, and neoisomenthol.

For example, in the method of preparing menthol according to an aspect, the selectivity of menthol ((menthol production amount/menthol isomer mixture production amount)x100) with respect to the menthol isomer mixture may be 30 % or more, or 40 % or more, or 50 % or more, and the upper limit thereof may be 95 % or less, or 90 % or less, or 80 % or less.

Hereinafter, the above-described implementation embodiment will be described in more detail through embodiments. However, the following embodiments are for explanation purposes only and do not limit the scope of rights.

### [Preparation Example] Preparing of Metal-supported Catalyst

40 g of porous silica powder having a surface area of 300 m²/g, a pore size of 21 nm, and an average particle size of 7 µm, 491 g of nickel sulfate (LiSO₄), 6 g of copper sulfate (CuSO₄), and 2,000 ml of distilled water were put in a precipitation vessel and stirred, and the temperature was raised to 80 °C. After reaching 80 °C, 1,500 mL of a solution including 262g of sodium carbonate (Na₂CO₃) was injected within 1 hour using a syringe pump. After completion of precipitation, pH of a slurry was 7.6, and the slurry was washed and filtered with about 30 L of distilled water and then dried at 100 °C for 12 hours or more using a drying oven. The dried material was divided into small parts, and then fired at a temperature of 350 °C in an air atmosphere. The fired material was divided into small parts again, and then reduced to a temperature of 350 °C in a hydrogen atmosphere to be activated. An activated catalyst was immobilized using a nitrogen mixture gas including 1 % of oxygen to prepare a nickel-silica supported catalyst.

### [Embodiment 1]

### Manufacture of Ammonia-treated Metal-supported Catalyst

The nickel-silica supported catalyst obtained in the Preparation Example was added to a reactor, and then the inside of the reactor was purged with nitrogen gas of 7.5 ml/min per g of catalyst. Thereafter, the reaction was treated with ammonia (NH₃) gas diluted to 10 % in helium (He) of 5 ml/min per g of catalyst for 30 minutes while maintaining the temperature at 20 °C, and then purged with nitrogen gas again to obtain an ammonia-treated nickel-silica supported catalyst. The processes were all performed under atmospheric pressure conditions.

### Preparation of Menthol

300g of thymol was added to an autoclave, the temperature of the autoclave was raised to 60 °C or less, when the thymol was melted, 4.5 g of the ammonia-treated nickel-silica supported catalyst obtained above was added and sealed. After sequentially purging with nitrogen and hydrogen, a reactor was filled with hydrogen up to 2 bars and stirred at 1600 rpm with a gas-induced hollow stirrer and heated to 160 °C. When the internal temperature of the reactor reached 160 °C, hydrogen was added at 10 bar and heated to 170 °C, and the reaction was performed while maintaining the temperature and pressure. Samples were collected at each reaction time and analyzed for residual thymol content, produced intermediate (menthone), menthol isomer, and hydrocarbon byproduct (menthane) content through gas chromatography (column: CP-ChiraSil-DEX CB (CP7502)). The results are shown in Table 1 below.

### [Embodiment 2]

The same process as Embodiment 1 was performed, except that the amount of catalyst used was changed to 18 g and the reaction temperature was changed to 180 °C.

### [Embodiment 3]

The same process as Embodiment 2 was performed, except that the reaction temperature was changed to 190 °C.

### [Comparative Example 1]

The same process as Embodiment 1 was performed, except that the nickel-silica supported catalyst obtained in the Preparation Example was used instead of the ammonia-treated nickel-silica supported catalyst.

**[Table 1]**

| | | **Comparative Example 1** | | **Embodiment 1** | | **Embodiment 2** | | **Embodiment 3** | |
|---|---|---|---|---|---|---|---|---|---|
| Reaction time (h) | | **2** | **3** | **2** | **3** | **1** | **3** | **0.5** | **1** |
| Thymol (wt%) | | 0.2 | 0.2 | 0.2 | 0.0 | 0.0 | 0.0 | 0.0 | 0.0 |
| Menthane (wt%) | | 4.5 | 4.4 | 3.6 | 2.3 | 2.7 | 2.5 | 3.8 | 3.6 |
| Menthol Isomers Mixtures (wt%) | Menthol | 26.5 | 28.7 | 27.6 | 32.9 | 43.1 | 54.1 | 44.6 | 54.7 |
| | Neomenthol | 13.6 | 14.4 | 15.4 | 16.3 | 20.9 | 25.4 | 22.1 | 26.2 |
| | Isomenthol | 49.0 | 45.5 | 44.0 | 42.8 | 29.6 | 15.6 | 25.7 | 13.3 |
| | Neoisomentol | 2.3 | 1.4 | 9.1 | 5.6 | 3.6 | 2.3 | 3.7 | 2.1 |
| Hydrocarbon byproducts (wt%) | | 3.9 | 5.4 | 0.1 | 0.1 | 0.1 | 0.1 | 0.1 | 0.1 |

Referring to Table 1 above, it may be seen that the preparing methods according to the embodiments have excellent reaction rates and simultaneously satisfy hydrocarbon byproducts production inhibition and menthol selectivity. On the other hand, the preparing method of Comparative Example was performed at the same reaction temperature as in Embodiment 1, but thymol was not completely converted within 3 hours, hydrocarbon byproducts increased significantly over time, and the selectivity of menthol was also significantly reduced compared to the embodiments.

While the present disclosure has been described with reference to the limited embodiments as described above, these are provided only to facilitate a more general understanding of the present disclosure. The present disclosure is not limited to the above embodiments, and various modifications and variations may be made from these descriptions by one of ordinary skill in the art to which the present disclosure pertains.

Therefore, the spirit of the present disclosure should not be limited to the described embodiments, and not only the claims described below, but also all those with modifications equal to or equivalent to the claims will fall within the scope of the spirit of the present disclosure.

## Claims

1. A method of preparing menthol, the method comprising preparing the menthol by a reduction reaction of thymol in the presence of an ammonia-treated metal-supported catalyst and hydrogen.

2. The method of claim 1, wherein
the metal-supported catalyst includes a nickel-supported catalyst including one or more active metals selected from nickel and nickel oxide.

3. The method of claim 2, wherein
the metal-supported catalyst includes a silica support.

4. The method of claim 1, wherein
treating the metal-supported catalyst with ammonia is performed with ammonia gas at a temperature of 300 °C or less.

5. The method of claim 1, wherein
the reduction reaction is performed under solvent-free conditions.

6. The method of claim 1, wherein
the reduction reaction is performed under conditions of 1 bar to 100 bar of hydrogen and at a temperature of 150 °C to 200 °C.

7. The method of claim 1, wherein
the reduction reaction is performed for 0.5 hours to 5 hours.

8. The method of claim 1, wherein
an amount of the metal-supported catalyst used is 0.01 % by weight to 10 % by weight with respect to a total weight of menthol produced.

9. The method of claim 1, wherein
a conversion rate of the thymol is 95 % or more.

10. The method of claim 9, wherein
a yield of a menthol isomer mixture is 95 % or more.

11. The method of claim 10, wherein
the menthol isomer mixture includes menthol, isomenthol, neomenthol, and neoisomenthol.

12. The method of claim 11, wherein
a selectivity of menthol with respect to the menthol isomer mixture is 50 % or more.
